# EUROPEAN PATENT APPLICATION

(11) **EP 2 422 830 A1**
(43) Date of publication of application: **29.02.2012**
(21) Application number: 10173658.5
(22) Date of filing: 23.08.2010
(51) Int. Cl.: A61M 5/142, B06B 1/02, G08B 3/10

(54) **Acoustic warning level optimization in ambulatory medical systems**

(71) Applicant: F. Hoffmann-La Roche AG, 4070 Basel (CH); Roche Diagnostics GmbH, 68305 Mannheim (DE)
(72) Inventor: Kalt, Lucas, 3053, Münchenbuchsee (CH); Remde, Axel, 3432, Lützelflüh (CH)
(74) Representative: Rentsch Partner AG

(57) **Abstract**

An ambulatory medical device (10) according to the invention comprises a function module (18) adapted to provide the intended functionality of the device, a controller module (24) adapted to control the device, and a sound generation module (11) with an acoustic transducer (16) adapted to produce an acoustic signal (56) and a signal generator (26) adapted to drive the acoustic transducer with a certain frequency, wherein the signal generation module (11) is arranged within a housing of the device. A tuning module (28, 30) is adapted to vary the frequency used by the signal generator to drive the acoustic transducer, and to determine one or more frequencies that correspond to a high sound level of the acoustic signal (56) outside of the housing of the device.

## Description

### Field of the Invention

The invention relates to ambulatory medical devices that provide acoustic warning signals, to methods to configure acoustic warning signal generation in such devices, and to devices for carrying out such a configuration.

### State of the art

Many medical devices are required to comply with strict requirements concerning providing warning signals to users and medical personnel that indicate potentially dangerous situations and incidents. Specifically, for medical devices that are intended to be carried by a user respectively patient during daily life, such as external ambulatory infusion devices and the like, regulatory requirements demand the provision of warning signals with a sound level over a minimum level, to ensure the audibility of the warning signal also in ambient noise situations.

External ambulatory infusion devices for the infusion of a liquid medicament over an extended time period are known in the art for a number of therapies. In particular, such devices form the basis for a state-of-the-art therapy of Diabetes Mellitus by CSII (Continuous Subcutaneous Insulin Infusion). An example of such an ambulatory infusion device is disclosed in WO 2003053498 A2 to which reference is made for a typical design and typical features of such devices.

Insulin infusion devices are designed to be carried by a user continuously, night and day, and to be concealed from view. They are intended to infuse insulin to the patient substantially continuously according to a time-varying schedule, and to infuse larger drug boli on demand. Besides diabetes therapy, these devices may be used for a number of further therapies, such as cancer treatment or pain therapy, without requiring substantial modification. Other examples of ambulatory medical devices are diagnostic systems for the continuous monitoring of physiological parameters, such as glucose level monitoring devices.

Such devices have to be robust in design and need to be protected from various environmental influences such as humidity and dirt. Advantageously they are waterproof. These goals are achieved by providing the devices by hermetically sealed housings. As a result the fully assembled devices do not comprise openings. A small aperture, with a diameter of for example 1 mm, is typically provided for pressure equalization purposes, which is covered by a gas-permeable but liquid-proof membrane. Such apertures, however, do not significantly influence the sound transfer from inside of the device to the outside.

Ambulatory medical devices typically comprise an acoustical transducer, for generating audible alarm signals and/or for providing user feedback. Especially for acoustic warning signals, a high sound level is generally desirable, to ensure audibility also in noisy environments. Regulatory requirements often define a minimum sound level that has to be reached. Norm ISO 60601-1-8 for example requires an acoustic warning sound level of at least 50 dB.

Ambulatory medical devices are generally equipped with batteries. Furthermore patients attach great importance to convenience and discretion. Thus such devices are required to have small dimensions and a low weight. Because the sound can only be transmitted from the acoustical transducer, which has to be located within the sealed device, to the environment via the housing walls, a considerable attenuation occurs. Given the general design constraints for such devices, in particular with respect to size and power consumption, as well as the necessary hermetical sealing of the device housing, the provision of a sufficient acoustic warning sound level is therefore a critical issue. For current devices it is known that alarms and acoustic warnings are sometimes not recognized if the medical device is in a pocket or the like, and there is a considerable ambient noise level.

Although modern production facilities allow the manufacture of medical devices and the basic components with high and constant quality, there will always be - within the defined tolerances - a remaining statistical spread of the characteristics of the single components as well as of the assembled device. Therefore certain operational parameters, such as for example the operational frequency of the acoustical transducer, have to be defined for an average case.

### Objects of the Invention

It is an object of this invention to provide a medical device, particularly, an ambulatory medical device that improve the state of the art with respect to the above-mentioned and other problems, and particularly allow the generation of acoustic warning signals with optimum sound volume. An optimum sound volume can be a optimum sound level achievable with a certain device, and/or a optimum sound level for a certain user.

Such ambulatory medical devices should be producible with high quality at reasonable costs, and should be robust and reliable. Preferably such an ambulatory medical device should be able to compensate aging effects, and should be adjustable to an individual user.

A further object of the invention is to provide a method for configuring the acoustic warning signal generation in ambulatory medical devices. Such a method should optimize the acoustic warning signal in regard to the perceivable sound level. Preferably such a method should take into account the statistical variability of individual devices and/or the hearing abilities of individual users.

Another object of the invention is to provide a device for configuring the acoustic warning signal generation in ambulatory medical devices.

These and other objects are achieved by an ambulatory medical device, a method for configuring the acoustic warning signal generation in ambulatory medical devices, and a device for configuring the acoustic warning signal generation in ambulatory medical devices, according to the independent claims. In addition, further advantageous embodiments follow from the dependent claims and the description.

### Summary of the invention

The present invention aims to individually optimize the sound level of acoustic warning signals for each ambulatory medical device, by tuning the frequency of the electrical signal that drives the acoustic transducer generating the warning signal to a value that results in a maximum perceivable sound volume.

An ambulatory medical device according to the invention comprises a function module adapted to provide the intended functionality of the device, a controller module adapted to control the device, and a sound generation module with an acoustic transducer adapted to produce an acoustic signal and a signal generator adapted to drive the acoustic transducer with a certain frequency, wherein the signal generation module is arranged within a housing of the device. A tuning module is adapted to vary the frequency used by the signal generator to drive the acoustic transducer, and to determine one or more frequencies that correspond to an optimum sound level of the acoustic signal. The optimum sound level of the acoustic signal is advantageously a maximum sound level emitted by the acoustic transducer, a maximum sound level as measured outside of the housing of the device, and/or a maximum sound level as determined by a user.

Advantageously the tuning module comprises one or more sensors for obtaining feedback data directly and/or indirectly related to the sound level of the acoustic signal outside of the housing.

In an advantageous embodiment of the invention the tuning module is adapted to measure an electric parameter of the acoustical transducer as a measure for the sound level generated by the transducer, e.g. the electric current drawn by the transducer or the impedance of the transducer.

In another advantageous embodiment of a device according to the invention the tuning module comprises a microphone adapted to measure a sound level outside of the housing of the device, an acceleration sensor adapted to measure an acceleration of a portion of the housing walls, a vibration sensor adapted to measure a vibration of a portion of the housing walls, and/or an optical distance measurement sensor adapted to measure a deflection of a portion of the housing walls.

In the ambulatory device according to the invention, the tuning module can be arranged fully or partially outside of the housing of the ambulatory medical device. In addition or alternatively the tuning module can be arranged fully or partially in a physically separate unit of the ambulatory medical device. In a particularly advantageous embodiment, such devices comprise an interface adapted to establish a data link between different parts of the tuning module.

An ambulatory medical device according to the invention can be an infusion pump device, particularly an insulin infusion pump device, or a device for monitoring the glucose level in the blood of a patient, or any other ambulatory medical device were an acoustical warning signal must be provided.

In a method according to the invention for configuring a sound generation module of an ambulatory medical device, a test frequency is determined; an acoustic signal is generated with the sound generation module, applying said test frequency; and a feedback signal is obtained and processed to a feedback value, wherein the feedback signal and value is related to the sound level of the acoustic signal outside of a housing of the device. These steps are repeated at least once for another test frequency. Based on the test frequencies and their corresponding feedback values, at least one optimum frequency is determined that corresponds to an optimum sound level of the acoustic signal. The optimum sound level of the acoustic signal is advantageously a maximum sound level emitted by the acoustic transducer, a maximum sound level as measured outside of the housing of the device, and/or a maximum sound level as determined by a user.

In an advantageous variant of such a method according to the invention, the test frequency is swept though a certain range, the feedback values are stored as a function of the test frequency, and the at least one optimum frequency is determined by identifying extremes in the feedback value function.

A configuration module according to the invention for configuring a sound generation module of an ambulatory medical device comprises a sensor for measuring a feedback signal corresponding to the sound level generated by a sound generation module of the device, a signal processing module for processing the feedback signal to a feedback value, and an interface for communicating with the ambulatory medical device.

An advantageous embodiment the configuration module comprises a controller module adapted to control the frequency applied for the sound generation module of the device via the interface. Alternatively or in addition the configuration module can be adapted to temporarily store feedback values, and to determine extreme values in the stored feedback values.

### Brief description of the drawings

In order to facilitate a fuller understanding of the present invention, reference is now made to the appended drawings. These references should not be construed as limiting the present invention, but are intended to be exemplary only.
- Figure 1: schematically shows an embodiment of an ambulatory medical device according to the invention, with an internal tuning module.
- Figure 2: shows a graph displaying the current drawn by an acoustic transducer of an ambulant medical device, as a function of the signal frequency, as well as the resulting measured sound level.
- Figure 3: schematically shows another embodiment of an ambulatory medical device according to the invention, operationally connected to an external tuning module.
- Figure 4: schematically shows a further embodiment of an ambulatory medical device according to the invention, with a user providing feedback for the internal tuning module.
- Figure 5: schematically shows yet another embodiment of an ambulatory medical device according to the invention, with an external feedback module.

### Description of embodiments of the invention

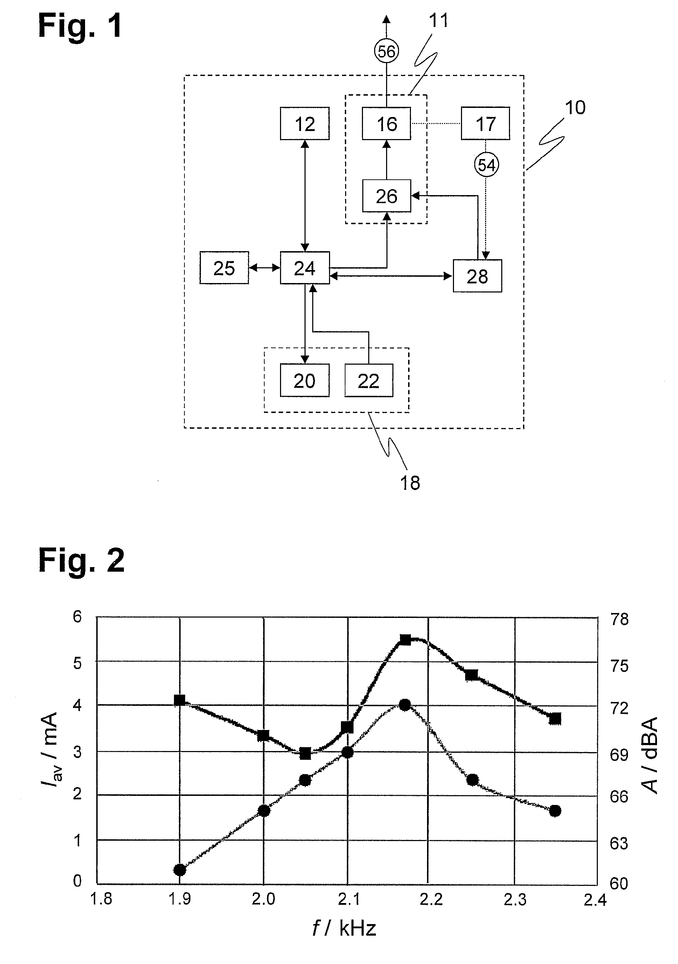

A first embodiment of an ambulatory medical device 10 according to the invention with an internal tuning module 28 is schematically shown in Figure 1. The ambulatory medical device 10 comprises a controller module 24, a function module 18, a sound generation module 11, and a user interface module 12.

In the disclosed embodiment the function module is the infusion pump module of an infusion pump device, however, the inventive principle is applicable to any ambulatory medical device with acoustic warning capabilities. The pump module 18 of the infusion pump device 10, comprises a drive system 20, one or more sensors 22, and other components that are not shown, since they are not relevant for the invention.

The controller module 24 is typically a microprocessor based controller. The controller module 24 operates the function module 18, and receives sensor data from the function module 18 to supervise the operation of the function module. The device can further comprise a data interface (not shown) for connecting to a remote control device, and/or a remote computer, and the like. Advantageously such a data interface is a wireless interface. "

A memory module 25 is operationally connected to the controller module 24, and is foreseen to store configuration data and/or data related to the operation of the device.

The user interface module 12 comprises one or more data input/output means that allows the user to receive information from the device 10, and to enter data or commands. The data input/output means can comprise, for example, a screen for optically presenting data, a keypad or buttons to manually enter data, or a touch screen.

Primarily, the sound generation module 11 is intended to deliver acoustic warning signals to the user of the device. In the case of an infusion pump device this warning can for example be related to a detected occlusion within the fluid system or another malfunction of the device, or an empty medicine reservoir. In addition it can be used to provide the user with acoustical feedback. The sound generation module 11 comprises at least one acoustical transducer 16, and a signal generator 26 providing an AC driving signal to the at least one transducer 16. The signal generator is controlled by the controller module 24, and generates a signal of sinusoidal, rectangular or any other wave form. A rectangular wave form is typically applied because due its simple technical implementation. The signal generator 26 can generate signals of variable frequency.

For the acoustic transducer 16 various designs are known in the art. Typically, it is a miniaturized dynamic loudspeaker or a piezo buzzer. Within the description of this invention, a tactile indicator, such as a vibration-generating device, is also understood to be an acoustic transducer 16, if it is driven by a AC signal.

For optimizing the acoustic alarm signal, particularly maximizing the sound level perceivable by the user, a tuning module 28 is provided. The tuning module 28, which is controlled by the controller module 24, is adapted to find the optimum operational frequency for the signal generator 26, which is correlated to a maximum objective sound level.

It should be noted that for the purpose of illustration and clarity the controller module 24, the memory module 25, the signal generator 26 and the tuning module 28 are shown as separate structural blocks. In a concrete embodiment, however, said modules can be realized as fully or partly integral electronic circuits. Advantageously they are part of the overall electronic circuitry board of the device.

At a certain optimum frequency that is individual for every particular ambulatory device, the sound level that is perceivable outside of the housing will assume a maximum value. Among other factors, said optimum frequency depends on the optimum operational frequency of the transducer 16, as well as the frequency dependent attenuation characteristics of the sealed housing of the device 10. To find this optimum operational frequency and to maximize the acoustic warning sound level, the sound level can be measured as a function of the signal frequency.

Since in an ambulatory device as shown in Figure 1 a direct measurement of the sound volume outside of the housing is prevented by the hermetical sealing, an alternative feedback signal inside of the device is used, which is correlated to said outside sound level. Which feedback signal is suitable and appropriately correlates to the sound level depends on the particular design of the ambulatory medical device, particularly the type of the acoustical transducer used. Advantageously the feedback signal should reach a maximum or minimum value at the resonance frequency of the transducer. For example, the electric current drawn by a transducer 16 during operation, or its electrical resistance, can be utilized to find the optimum frequency for the transducer operation.

Figure 2 exemplarily shows the sound level (filled circles) of a miniaturized dynamic loudspeaker as the transducer, along with the current (filled squares) drawn by the loudspeaker, as a function of the applied frequency. The loudspeaker is arranged in a closed housing, with the sound level being measured outside the housing. As can be seen, for this setup the drawn current assumes a maximum at the maximum sound level. Therefore the sound level and the drawn current correlate; the current is a suitable feedback parameter.

The feedback parameter cannot take into account a frequency dependent attenuation of the sound signal by the housing. To compensate this effect, an average attenuation curve of that particular type of housing can be applied to correct the optimum frequency. If the average attenuation of a housing type is sufficiently frequency-independent in the relevant frequency range, the housing attenuation may remain unconsidered for practical purposes.

In the device as shown in Figure 1, the following procedure is carried out to configure the sound generation module 11: The tuning module 28 initiates a tuning process, by activating the sound generation module 11, such that an acoustical alarm signal 56 is generated by the acoustic transducer 16, with a certain frequency delivered by the signal generator 26. The tuning module 28 receives a feedback signal 54 from a sensor 17, wherein the feedback signal is correlated to the sound level generated by the transducer 16. The tuning module 28 changes the frequency used by the signal generator 26 to drive the transducer 16, according to a certain pattern. The tuning module 29 can for example increase the frequency stepwise or continuously, thereby sweeping through a defined frequency range. Alternatively, a limited set of frequencies within a defined range can be tested, etc.

During variation of the signal frequency, the tuning module 28 compares the feedback signal 54 with the previously obtained feedback values, in order to find an maximum (or minimum, depending on the used feedback signal), that can be correlated to a maximum sound level of the acoustic transducer. Once the extreme value is determined, the frequency variation can be stopped. The tuning module 28 delivers the determined optimum frequency to the controller module 24, which stores the information in the configuration memory 25, to be used for alarm signal generation in the future. The configuration of the sound generation module is completed.

The configuration procedure can be initiated in a number of ways. For example, the tuning process be carried out in the production facility as part the general testing and configuration procedures following the device manufacture. Alternatively or additionally, the device user or a maintenance person may initiate the configuration procedure at their discretion. Alternatively or additionally, the configuration procedure can be initiated automatically on suitable occasions, for example each time a device battery is replaced, or a drug cartridge or another disposable component is changed. Such a repetition of the tuning process after production allows compensating aging effects and the like.

Since an internal tuning module 28 can be realized with limited additional hardware and firmware effort, it can be implemented in the ambulatory medical device at reasonable costs. An internal tuning module allows carrying out the optimization whenever required, as described above.

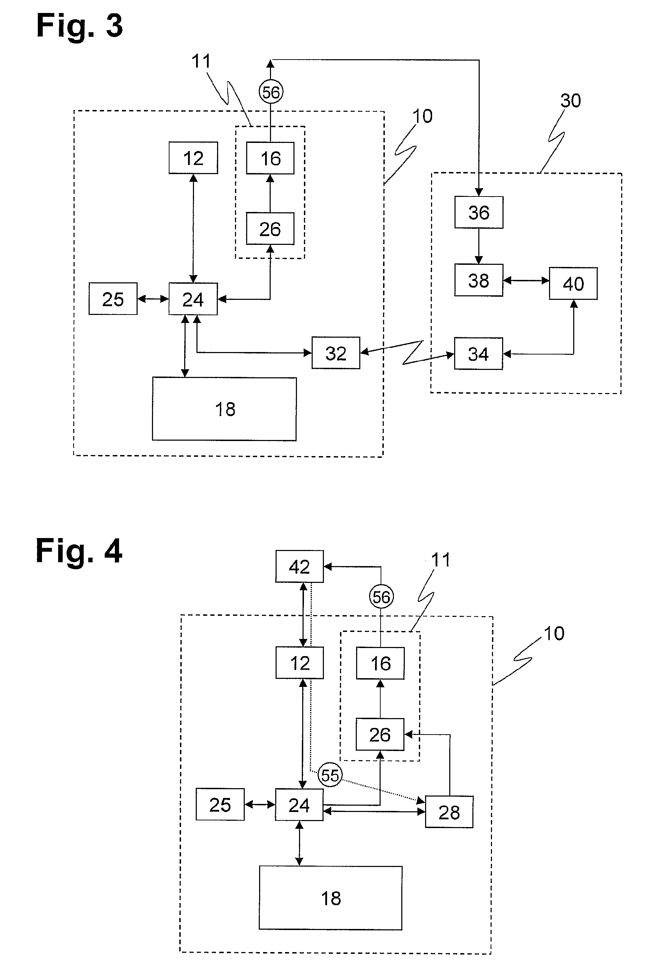

Figure 3 shows an alternative embodiment of an ambulatory medical device 10 according to the invention that is especially advantageous for use in the context of device configuration and setup at a production facility.

An external tuning module 30 is provided that is temporarily operationally connected to the ambulatory medical device 10 according to the invention, in order to tune the signal frequency to an optimum with maximum perceivable sound level. For that purpose the ambulatory medical device 10 is provided with a second interface 32, which is adapted to communicate with a corresponding interface 34 of the external tuning module 30. Said second interface 32 may be particularly dedicated to this purpose, or it can be identical with an interface of the device 10 adapted to communicate with a remote control device. The latter variant has the advantage that no additional electronic components are required.

Furthermore the external tuning module 30 comprises a sensor module 36 that is adapted to directly or indirectly measure the sound energy generated by the acoustic transducer 16 that actually arrives outside. A signal processing module 38 processes the data received by the sensor module 36 to sound level data, and a controller module 40 assesses the determined sound level data.

Via the interface 32, 34 the controller module 40 of the external tuning module 30 can instruct the sound generation module 11 of the ambulatory medical device 10 to activate and deactivate the acoustic transducer 16, and to change the operational frequency of the signal generator 26.

The sensor module 36 can for example be realized as a microphone, advantageously located in a defined position in regard to the device, to directly measure the sound level outside of the housing. The sound level can also be measured indirectly by determining the acceleration or the amplitude of deflection of certain parts of the housing walls, by an acceleration or vibration sensor that is mechanically coupled to the housing of the device, or an optical distance measurement sensor that is adapted to measure the deflection of one or more defined portions of the housing.

The signal processor module 38 typically includes amplifiers, filters, analogue-to-digital conversion circuitry, and the like. The signal processor module 38 can further include an nonlinear circuitry, in particular a logarithmic amplifier and an A-curve or C-curve filter, for simulating human hearing characteristics.

The interface 34, 32 between external tuning module and ambulatory medical device 10 is advantageously a wireless interface. It can be realized, for example, as an infrared interface or a short range radio frequency interface, e.g. BlueTooth® (IEEE 802.15.1).

Via the data link established by interface 32, 34 the controller module 40 of the external tuning device instructs the controller module 24 of the ambulatory medical device 10 to change the frequency generated by signal generator 26. As in the previously described embodiments, a certain frequency range may be swept, while sampling the sound level data. In this case, the optimum frequency is advantageously determined after completing the sweep through the frequency range. The resulting optimum frequency is then communicated to the controller module 24 of the ambulatory medical device 10, and stored in the configuration memory 25 for later use. Alternatively, a stop signal may be transmitted to the ambulatory medical device at the maximum sound level, thus stopping the sweeping.

The external tuning module 30 can either be realized as a stand-alone configuration device or as an integral part of a larger testing and/or configuration station, which is used for testing further functions of the infusion device, writing configuration data into the device memory, or the like.

The advantage of the embodiment shown in Figure 3 is the measurement of the relevant parameter, namely the achievable sound level outside of the housing. Thus, not only the variability of individual resonance frequencies of the used acoustic transducer components can be taken into account, but also individual resonance frequencies of the housings, as well as the mechanical coupling between the transducer 16 and the housing, etc.

In the variant discussed above, the configuration procedure as such is controlled and monitored by the external tuning module 30, sending instructions to the controller module 24 of the ambulatory device 10. Alternatively, however, the main control of the configuration procedure can be assigned to the ambulatory device 10 instead, namely to an internal tuning module 28. In such a variant the external module with the sensors has the primary function of providing the internal tuning module with the received sound level data either in raw form or already processed, while the optimum frequency is determined internally.

In a further variant, the internal tuning module controls the frequency variation, while the external module returns the determined optimum frequency to the internal tuning module.

Yet another advantageous embodiment of an ambulatory medical device according to the invention is depicted in Figure 4. In this embodiment, a device user 42 assesses the alarm sound level. After the user has initiated the configuration procedure, the tuning module 28 varies the frequency used by signal generator 26, for example by sweeping through a certain frequency range. The user 42 provides feedback of the perceived sound level via the user interface module 12 to the tuning module 28, which subsequently determines an optimum frequency based on said user feedback. Thus such an embodiment of the invention allows optimizing the subjective sound level for a specific user, in contrast to the preceding embodiments, where the objective sound level, the measurable physical quantity, is optimized.

Since it is not possible for humans to exactly quantify a perceived sound level, in an advantageous variant of the invention the controller module 24 is adapted to present to the user a restricted number of acoustic signals with different frequencies. The user then tags these alarm signals with a perceived qualitative sound level. For example the user 42 can sort the acoustic signals in the order of increasing or decreasing sound volume. In the simplest case of only two signals with different frequencies, for example, the user informs the tuning module which signal he perceived to be louder. After collecting sufficient feedback data 54 from the user, the controller/tuning module can then determine the optimum frequency for the acoustic alarm signal.

In a particularly advantageous variant of the invention, the controller module 24 will continuously adapt the frequencies presented to the user 42, based on the already obtained feedback data, in order to more efficiently determine the optimum frequency.

In a further variant the signal generator slowly sweeps through a frequency range, as long as the user continues to press a defined button or the like of the user interface module 12, and stops sweeping when the user releases the button. The user will press the button as long as the perceived sound level increases, and will release the button when the perceived sound level decreases. This optimum frequency is then stored in the memory 25 as the acoustic alarm frequency. The process may also be carried out in two steps, including a first coarse tuning, and a subsequent second fine tuning.

The advantage of the variant of the invention as presented in Figure 4 is that the optimum frequency is adapted to a specific, individual person, namely the user 42 carrying the ambulatory medical device. Thus the determined frequency takes into account not only the relevant parameters related to sound generation and transmission, particularly the resonance frequencies of the acoustic transducer and the device housing. In addition also the personal characteristics of the physiological sound perception of a specific person are considered. The user may have for example reduced or increased hearing capabilities in certain frequency ranges. Thus hardness of hearing or other user specific issues are automatically compensated. Furthermore such a device requires only minimal hardware and software effort.

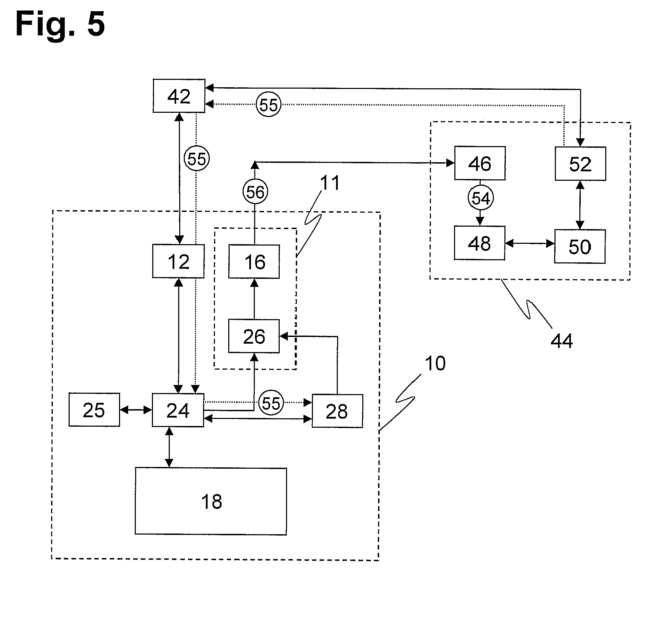

Yet another embodiment of an ambulatory medical device according to the invention is described in Figure 5, with an external feedback module 44 measuring the sound level. Af ter the user 42 has activated the external feedback module 44 and has initiated the tuning procedure via the interface 12, the internal tuning module 28 changes the frequency used by the signal generator 26 according to a defined pattern, e.g. a sweep.

A sensor module 46 of the external feedback module 44 receives the sound signal 56 produced by the acoustic transducer 16. A signal processing module 48 determines the sound level, and the controller module 50 communicates the found sound level value in a suitable form to the user 42, via a user interface module 52, e.g. a screen. The user reads the presented value and enters the value into the ambulatory medical device 10, using the user interface module 12.

The particular advantage of this variant is the fact that on one hand the sound level can be exactly quantified, and that on the other hand no additional interface is necessary. The user himself transmits the feedback data 54 from the external feedback module 44 to the ambulatory medical device 10. Such an external feedback module 44 can be manufactured at comparably low costs, since as a minimum it essentially has to comprise only a microphone, an on/off button, a few LEDs, and inexpensive integrated circuits.

Such an embodiment is particularly useful for example for first level support maintenance personnel, or even the user himself.

In a particularly advantageous embodiment of the invention, an external tuning module or an external feedback module can be realized as an integrated part of a remote control module that is intended to wirelessly communicate with the ambulatory medical device.

An advantageous device according to the invention can be adapted to generate, during operation, signals of different frequencies, for example for distinguishing between alarms of primary importance that require an immediate action, and further warnings or reminders of secondary importance. For such an embodiment, the above-defined procedure may be carried out separately for the different types of signals. The frequency range available for the different acoustic signal types should be sufficiently, such that the frequency ranges do not overlap and the signals are clearly identifiable.

Besides the feedback means that have been discussed so far, all thinkable kinds of feedback can be applied whenever it leads to an desirable optimization of the acoustic signal.

The present invention is not to be limited in scope by the specific embodiments described herein. Indeed, various modifications of the present invention, in addition to those described herein, will be apparent to those skilled in the art from the foregoing description and accompanying drawings. Thus, such modifications are intended to fall within the scope of the appended claims. Additionally, various references are cited throughout the specification, the disclosures of which are each incorporated herein by reference in their entirety.

### List of Reference Numerals

- 10: ambulatory medical device
- 11: sound generation module
- 12: user interface module
- 16: acoustical transducer
- 17: sensor
- 18: function module, pump module
- 20: drive system
- 22: sensor
- 24: controller module
- 25: memory
- 26: signal generator
- 28: internal tuning module
- 30: external tuning module
- 32: interface
- 34: interface
- 36: sensor module
- 38: signal processing module
- 40: controller module
- 42: user
- 44: external feedback module
- 46: sensor module
- 48: signal processing module
- 50: controller module
- 52: user interface module
- 54: feedback signal
- 55: feedback value
- 56: acoustic warning signal
- 70: sound level
- 72: current

## Claims

1. An ambulatory medical device (10), comprising a function module (18) adapted to provide the intended functionality of the device, a controller module (24) adapted to control the device, and a sound generation module (11) with an acoustic transducer (16) adapted to produce an acoustic signal (56) and a signal generator (26) adapted to drive the acoustic transducer with a certain frequency, wherein the signal generation module (11) is arranged within a housing of the device (10), **characterized by** a tuning module (28, 30) that is adapted to vary the frequency used by the signal generator to drive the acoustic transducer, and to determine one or more frequencies that correspond to an optimum sound level of the acoustic signal (56).

2. The device according to claim 1, **characterized in that** the optimum sound level of the acoustic signal (56) is a maximum sound level emitted by the acoustic transducer (16), a maximum sound level as measured outside of the housing of the device (10), and/or a maximum sound level as determined by a user (42).

3. The device according to claim 1 or 2, **characterized in that** the tuning module (28, 30) comprises one or more sensors (17, 36, 46) for obtaining feedback data (54) directly and/or indirectly related to the sound level of the acoustic signal (56) outside of the housing.

4. The device according to any of the preceding claims, **characterized in that** the tuning module (28) is adapted to measure an electric parameter of the acoustical transducer (16) as a measure for the sound level generated by the transducer, e.g. the electric current drawn by the transducer or the impedance of the transducer.

5. The device according to any of the preceding claims, **characterized in that** the tuning module (30) comprises a microphone adapted to measure a sound level outside of the housing of the device (10), an acceleration sensor adapted to measure an acceleration of a portion of the housing walls, a vibration sensor adapted to measure a vibration of a portion of the housing walls, and/or an optical distance measurement sensor adapted to measure a deflection of a portion of the housing walls.

6. The device according to any of the preceding claims, **characterized in that** the tuning module is arranged fully or partially outside (30, 44) of the housing of the ambulatory medical device (10).

7. The device according to any of the preceding claims, **characterized in that** the tuning module is arranged fully or partially in a physically separate unit (30, 44) of the ambulatory medical device (10).

8. The device according to claim 6 or 7, **characterized by** an interface (32, 34, 12, 52) adapted to establish a data link between different parts of the tuning module.

9. The device according to any of the preceding claims, **characterized in that** the device (10) is an infusion pump device, particularly an insulin infusion pump device, or a device for monitoring the glucose level in the blood of a patient.

10. A method for configuring a sound generation module (11) of an ambulatory medical device (10), wherein
(a) a test frequency is determined (28, 40);
(b) an acoustic signal (56) is generated with the sound generation module (11), applying said test frequency;
(c) a feedback signal (54) is obtained (17, 36, 46) and processed (28, 38, 40, 48, 50) to a feedback value (55), wherein the feedback signal and value is related to the sound level of the acoustic signal (56) outside of a housing of the device (10);
(e) the steps (a) to (d) are repeated at least once for another test frequency; and
(f) based on the test frequencies and their corresponding feedback values, at least one optimum frequency is determined that corresponds to a optimum sound level of the acoustic signal (56).

11. The method according to claim 10, **characterized in that** the optimum sound level of the acoustic signal (56) is a maximum sound level emitted by the acoustic transducer (16), a maximum sound level as measured outside of the housing of the device (10), and/or a maximum sound level as determined by a user (42)

12. The method according to claim 10 or 11, **characterized in that** the test frequency is swept though a certain range, the feedback values are stored as a function of the test frequency, and the at least one optimum frequency is determined by identifying extremes in the feedback value function.

13. A configuration module (30, 44) for configuring a sound generation module (11) of an ambulatory medical device (10), **characterized in that** the configuration module (30, 44) comprises a sensor (36, 46) for measuring a feedback signal (54) corresponding to the sound level generated by a sound generation module (11) of the device (10), a signal processing module (38, 48) for processing the feedback signal (54) to a feedback value (55), and an interface (34, 52) for communicating with the ambulatory medical device (10).

14. The configuration module according to claim 13, **characterized in that** the module (30) comprises a controller module (40) adapted to control the frequency applied for the sound generation module (11) of the device (10) via the interface (34).

15. The configuration module according to claim 13 or 14, **characterized in that** the module (30) is adapted to temporarily store feedback values (55), and to determine extreme values in the stored feedback values (55).
